# EUROPEAN PATENT SPECIFICATION

(11) **EP 0 829 236 B1**
(45) Date of publication and mention of the grant of the patent: **25.07.2001**
(21) Application number: 97120309.6
(22) Date of filing: 13.09.1993
(51) Int. Cl.: A61B 17/12, A61F 2/06

(54) **Detachable embolic coil assembly**
Anordnung einer lösbaren Emboliespiralfeder
Ensemble spirale embolique détachable

(30) Priority: 22.09.1992 US 949094; 19.04.1993 US 49577
(43) Date of publication of application: 18.03.1998
(62) Divisional of application: 93921506.7
(73) Proprietor: Target Therapeutics, Inc., Fremont, CA 94538 (US)
(72) Inventor: Palermo, Thomas J., San Jose, California 95124 (US)
(74) Representative: Price, Nigel John King

(56) References cited:
- WO-A-92/14408

## Description

This invention is a surgical instrument and specifically is a device for delivering embolic coils to a selected site within the vasculature or other lumen of a human body via use of a catheter. The invention further includes the coils. In particular, the device (typically a "pusher assembly" in conjunction with a catheter) uses embolic coils having interlocking ends, e.g., clasps or hooks, on at least one end of the coils. The coils may further be secured to each other by a control wire within the catheter. The coils are pushed out the end of the catheter for placement and retraction of the optional control wire into the catheter body uncouples the distal coil. If no control wire is used, the coil is self-disengaging.

The endovascular treatment of a variety of vascular maladies throughout the body is an increasingly more important form of therapy. Catheters have been used to place various treatment materials, devices, and drugs within arteries and veins in the human body. Examples of these devices and their use in such treatments are shown in US-A-5,234,437 ("Detachable Pusher-Vasoocclusive Coil Assembly with Threaded Coupling") and US-A-5,261,916 ("Detachable Pusher-Vasoocclusive Coil Assembly with Interlocking Ball and Keyway Coupling"). These show methods and devices for delivery of coils or wires within the human body to sites such as aneurysms, to occlude those sites. Coils such as are discussed in US-A-4,994,069, may be of a regular or helical configuration or assume a random convoluted configuration at the selected site. The coils normally are made of a radiopaque, biocompatible metal such as platinum, gold, tungsten, or alloys of these and other metals.

In treating aneurysms it is common to place a number of coils within the aneurysm. The coils occlude the site by posing a physical barrier to blood flow and by promoting thrombus formation at the site.

Coils have typically been placed at the desired site within the vasculature using a catheter and a pusher. The site is first accessed by the distal end of a catheter. In treating peripheral or neural conditions requiring occlusion, the sites are accessed with flexible, small diameter catheters such as those shown in US-A-4,739,768 and US-A- 4,813,934. The catheter may be guided to the site through the use of guidewires (see US-A-4,884,579) or by flow-directed means such as balloons placed at the distal end of the catheter. Use of guidewires involves the placement of relatively long, torqueable proximal wire sections within the catheter, which proximal sections are attached to more flexible distal end wire section designed to be advanced across sharp bends at vessel junctions. The guidewire is visible using x-ray and allows a catheter to be manipulated through extremely tortuous vessels, even when such vessels are surrounded by soft tissue such as the brain.

Once the selected site has been reached, the catheter lumen is cleared by removing the guidewire (if a guidewire has been used), and the coil is placed into the proximal open end of the catheter and advanced through the catheter with a pusher. Pushers are wires having a distal end that is adapted to engage and push the coil through the catheter lumen as the pusher is advanced through the catheter. When the coil reaches the distal end of the catheter, it is discharged from the catheter by the pusher into the vascular site. This technique of discharging the coil from the distal end of the catheter has a number of undesirable limitations. First, because of the plunging action of the pusher and the coil, the positioning of the coil at the site cannot be controlled to a fine degree of accuracy. Second, once the coil has left the catheter, it is difficult to reposition or retrieve the coil if such is desired. Nevertheless, the technique has the benefit of delivering multiple coils at low cost with a short delivery time.

Several techniques have been developed to enable more accurate placement of coils within a vessel. In one technique (US-A- 5,122,136, on which the preamble of claim 1 is based) the coil is bonded via a metal-to-metal joint to the distal end of the pusher. The pusher and coil are made of dissimilar metals. The coil-carrying pusher is advanced through the catheter to the site and a low electrical current is passed through the pusher-coil assembly. The current causes the connection between the pusher and the coil to be severed via electrolysis. The pusher may then be retracted leaving the detached coil at an exact position within the vessel. In addition to enabling more accurate coil placement, the electric current may facilitate thrombus formation at the coil site. The only perceived disadvantage of this method is that the electrolytic release of the coil requires a period of time so that rapid detachment of the coil from the pusher does not occur.

Another technique for detaching an embolic coil is shown in US-A-5,261,916. In that document, a coil having an enlarged portion is mated with a pusher having a keyway adapted to receive the enlarged portion of the coil in an interlocking relationship is covered by a coaxial member about the pusher and the coil. The coaxial member is movable by sliding the member axially. As the coaxial member is moved away from the junction where the coil's member engages the member of the keyway of the pusher, the coil disengages and the pusher is removed.

Another device for placement of coils is shown in US-A-5,234,437. This device includes a coil having a helical portion at one end and a pusher which is threaded to the inside of the helical coil by the use of a threaded section on the outside of the pusher. The device operates to release the coil by engaging the proximal end of the coil with a sleeve while the pusher is unthreaded. Once the pusher is free, the sleeve may be used to push the coil out into the treatment area.

Another method of placing an embolic coil is shown in US-A-5,108,407. This patent shows the use of a device in which embolic coils are separated from the distal end of a catheter by the use of heat-releasable adhesive bonds. The coil adheres to the therapeutic device via a mounting connection using a heat sensitive adhesive. Laser energy is transferred through a fiber optic cable,' which cable terminates at the connector. The connector becomes warm and releases the adhesive bond between the connector and the coil.

None of these disclosed devices suggest coils having interlocking ends which allow an embolic coil to be positioned within a vessel and then released upon ejection of the coil from the catheter distal end or, optionally, upon retraction of a control wire positioned within that interlocking end.

According to the present invention there is provided a detachable coil assembly for use in occluding a selected site within a vessel, comprising a coil adapted for detachment in a body cavity and with opposing ends on a coil axis, characterised in that the coil has, on at least one end thereof, an interlockable, shaped clasp extending in the same general direction as the coil axis, said interlockable clasp being configured to interlock detachably with a similar clasp.

Embodiments of apparatus will now be described, by way of example only, with reference to the accompanying drawings, described below.

Figures 1A and 1B show, respectively, a partial sectional view of a pusher assembly and an engaged coil assembly having an interlocking clasp at only one end and a front three-quarters view of one variation of the interlocking clamp.

Figure 2 shows a series of coil assemblies having either one or two interlocking clasps at their ends.

Figure 3 shows deployment of the interlocking coil within a catheter.

Figures 4 and 5 show the operation of the assembly as it places a coil within a target site.

Figures 6A and 6B show, respectively, a partial sectional view of a pusher assembly and an engaged coil assembly having a variation of an interlocking clasp and a front three-quarter view of that variation of the interlocking clasp.

Figures 7A, 7B, and 7C show a method of attaching coils having the interlocking clasp shown in Figures 6A and 6B to a pusher body within the catheter lumen.

Figure 8 shows a variation of the invention in which both the coils and the pusher body have simple loops as interlocking clasps.

Figure 9A shows a side view of a clasp, joinable to a coil or pusher, similar in shape to the Figure 1A to 5 clasp but, in design without, without the control wire.

Figure 9B shows a front three-quarter view of the Figure 9A clasp.

Figure 10A shows a side view of a clasp similar to the clasp shown in Figures 9A and 9B but with a ramped end.

Figure 10B shows a front three-quarter view of the Figure 10A clasp.

Figure 11 shows the Figure 9A clasp mounted on a pusher and on a coil as would be seen in a catheter.

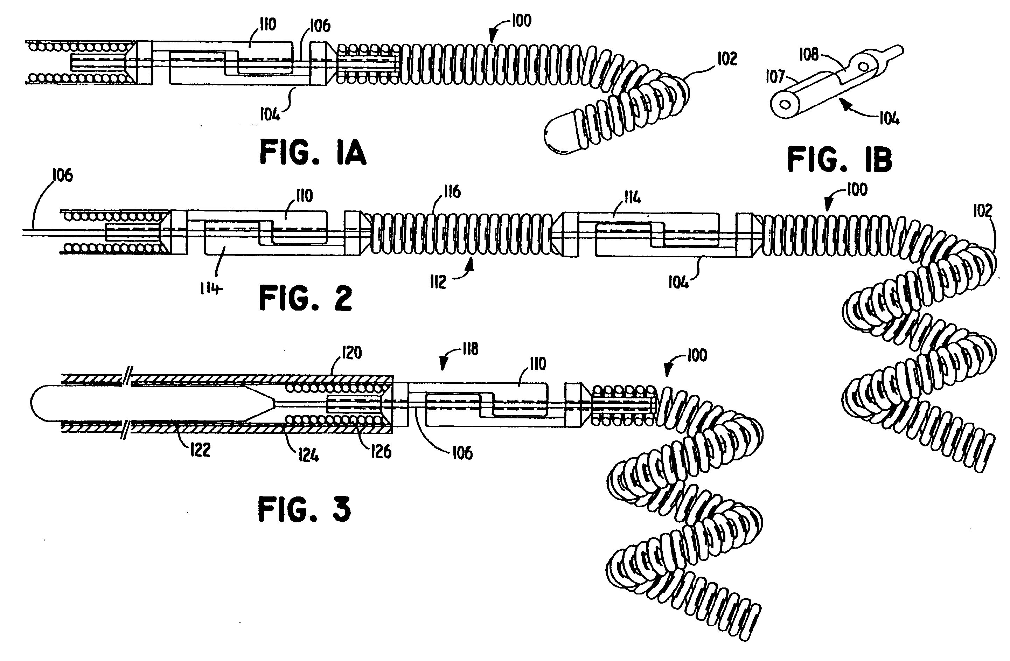

The coil assembly (100) is shown in Figure 1. The coil (102) is shown as helical in form, although it may be any other suitable form. The coil should be of a size sufficiently small that it may be advanced through a catheter that is appropriately sized for accessing the targeted vascular site. For instance, when accessing a brain aneurysm in a small vessel, an appropriately sized catheter is quite small and very flexible. The coil in such a situation must be small enough to fit through the catheter and out its distal end at the treatment site.

The coil is desirably made up of a radiopaque, physiologically compatible material. For instance, the material may be platinum, gold, tungsten, or alloys of these. Certain polymers are also suitable as coil material either alone or in conjunction with metallic markers providing radiopacity. These materials are chosen so that the procedure of locating coils within the vessel may be viewed using radiography. However, it is also contemplated that these coils may be made of various other biologically inert polymers or of carbon fiber.

The size of the coil and its constituent winding will depend upon the use to which the coil will be placed. For occluding peripheral or neural sites, the coils will typically be made of 0.05 to 0.15 mm diameter wire (platinum or platinum/tungsten alloy) that may be wound to have an inner diameter of 0.15 to 1.5 mm with a minimum pitch -- that is to say that the pitch is equal to the diameter of the wire used in the coil. The outer diameter is then typically between 0.25 mm to 1.8 mm. The length of the coil will normally be in the range of 0.5 to 60 cm, preferably 0.5 to 40 cm.

If desired, the coil may be formed in such a way that the coil is essentially linear as it passes through the catheter and yet assumes a randomly oriented relaxed condition after it is released from the distal end of the catheter. A discussion of this variation may be found in US-A-4,994,069.

Fixedly attached to coil (102), as is shown in Figure 1A, is interlocking clasp (104). Interlocking clasp (104) as is depicted in the front three-quarter view in Figure 1B, has an interior passageway allowing the control wire (106) to pass completely therethrough. As is shown in Figure 1A, the male portion of the next adjacent interlocking clasp (110) fits into the area (108) left within clasp (104) so to allow the interlocking to take place. Said another way, the distal portion of interlocking clasp (104) is generally cylindrical in shape but has a surface (107), which may be cut or milled away, allowing the portion to mesh within the middle area (108) of an adjacent clasp. The proximal section is adapted for attaching to a coil or to a pusher assembly. The attachment may be by welding, soldering, gluing, or the like. With a control wire (106) passing through the axis of both interlocking clasps (104) and (110), the two are locked together. As is shown in Figure 1A, the control wire may extend through the length of coil (102).

Figure. 2 shows an intermediate coil assembly (100) comprising coil (102) and interlocking clasp (104) (joined with coil assembly (112)) which has interlocking clasp (114) fixedly attached at both ends of the intervening coil (116). As was the situation in Figure 1A, the proximal interlocking clasp (114) is joined by control wire (106) with interlocking clasp (110). In this way, a significant number of coils (112) may be loaded onto a control wire (106) and delivered to the treatment site without removal of the control wire from the catheter.

Figure 3 shows the relationship of coil assembly (100) and the pusher assembly (118) with its distal interlocking clasp (110) as it fits within catheter sheath (120). Also shown is movable inner core member (122) and the sheath (124) which fits within catheter sheath (120) and supports interlocking clasp (110). Shown in Figure 3 is the stiffener spring (126) which provides form and support for the distal end of the pusher assembly (118) and in particular rigidly adheres to interlocking clasp (110). Inner core member (122) allows the control wire (106) to be moved axially along the interior of the catheter sheath (120) and the pusher assembly (118). Movement of the inner core member (122) in a proximal direction permits uncoupling of the coil as will be discussed in more detail below.

The length of pusher assembly (118) will be such as to be capable of being advanced entirely through the catheter to place coil (102) at the target site but yet with a sufficient portion of the proximal end of the pusher assembly (118) protruding from the proximal end of the catheter to enable the control wire (106) to be manipulated. For use in peripheral or neural surgeries, the pusher will normally be about 100-200 cm in length, more normally 130-180 cm in length. The diameter of the pusher assembly (118) is usually in the range of 0.25 to about 0.90 mm.

As indicated previously, conventional catheter insertion and navigational techniques involving guidewires or flow-directed devices may be used to access the site with a catheter. Once the distal end of the catheter is positioned at the site, often by locating its distal end through the use of radiopaque marker material and radiography, the catheter is cleared. For instance, if a guidewire has been used to position the catheter, it is withdrawn from the catheter and then the pusher assembly (118) having coil assembly (100) at the distal end is advanced through the catheter. The pusher assembly (118) is advanced past the distal end of the catheter so that the coil is free of the catheter and with the coil positioned precisely at the desired treatment site.

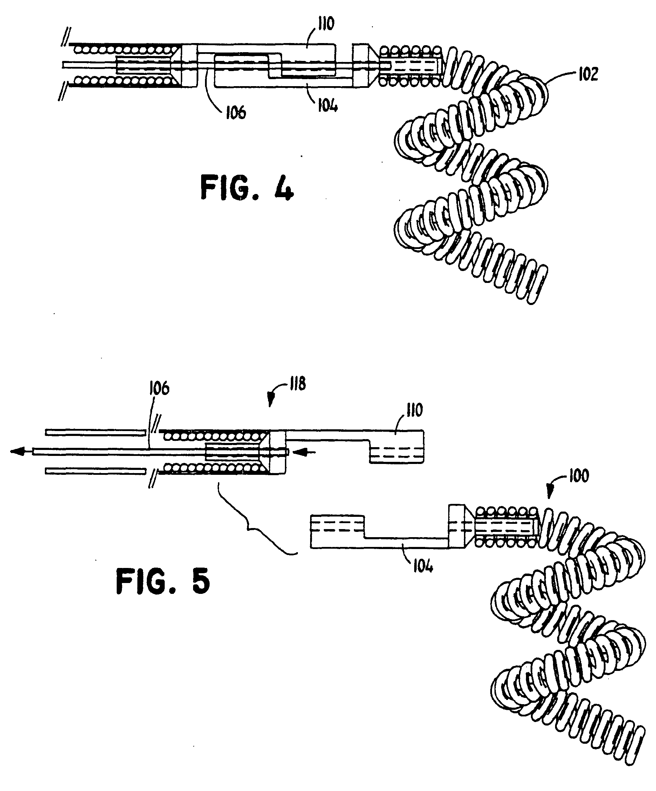

As is shown in Figures 4 and 5, control wire (106) is withdrawn from the junction between coil interlocking clasp (104) and the other interlocking clasp (110). Coil assembly (100) is then free. The entire catheter may then be removed or the pusher assembly (118) may be withdrawn from the catheter lumen to provide for installation of other coils. If additional coils are to be placed at the target site, the procedure is repeated. After the desired number of coils have been placed at the site, the catheter is withdrawn from the vessel.

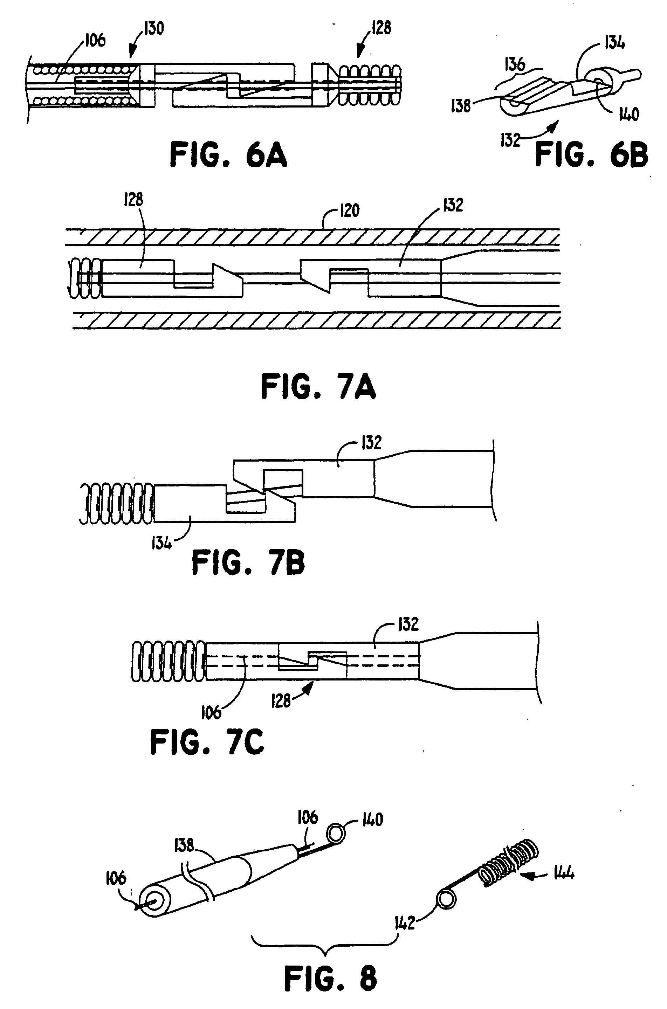

Figure 6A shows a variation in which coil assembly (128) is interlocked with pusher assembly (130) by control wire (106). The depicted coil assembly (128) and pusher assembly (130) are different in that they incorporate the interlocking clasp (132) design shown more clearly in Figure 6B. The interlocking clasp (132), as with the clasp depicted in Figure 1B, utilizes an open area (134) within the clasp (132) to accept the mating ramp latch (136) from another similar clasp. The ramp latch (136) typically has a slot (138) and a passageway (140) to permit passage of the control wire through the clasp (132) from end to end without obstruction.

The ramp latch (136) allows easy assembly of a string of coils within the catheter for subsequent placement using the device.

Such an assembly process is shown in Figures 7A, 7B, and 7C.

Figure 7A shows a pusher assembly (130) approaching a coil assembly (128) which has been previously placed within a catheter sheath (120). The distal interlocking clasp (132) on the pusher assembly (130) is positioned to interlock with the proximal interlocking clasp (134) on the coil assembly (128).

Figure 7B shows the two interlockng clasps (132 and 134) as they approach their respective ramps contacting and causing the two clasps to displace axially within the catheter sheath.

Figure 7C shows the location of the coil assembly (128) and the pusher assembly (132) after the respective clasps are interlocked and the control wire (106) has been placed through the passageways within the clasps.

Figure 8 shows an elegantly simple variation of the invention in which the pusher (138) is a tubing member having a control wire (106) within its core. The clasp portion (140) is a simple loop comprising, e.g., wire or small rod. The corresponding interlocking loop (142) on the coil (144) forms the junction with the clasp on the pusher.

The variation of the invention shown in Figures 6A, 6B, 7A, 7B, 7C, and 8 may be placed within the vasculature in the same manner as shown for the variation shown in Figs. 4 and 5.

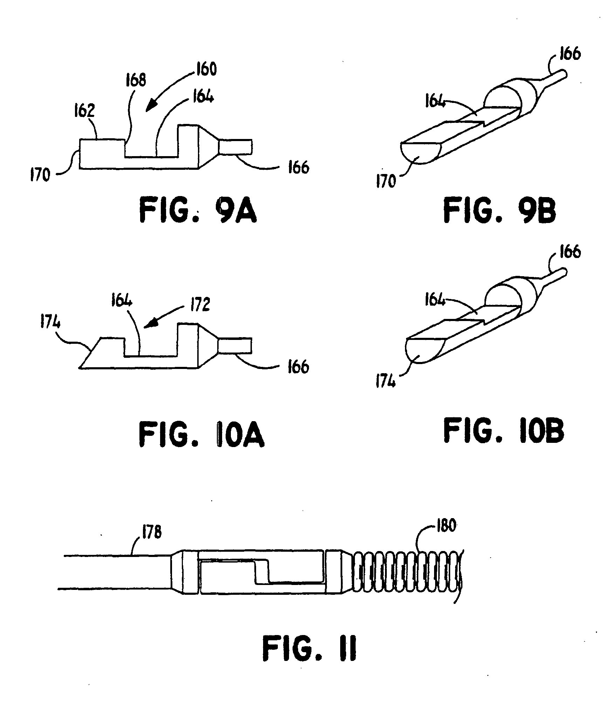

Figure 9A shows a side view of a clasp (160) similar in design to the clasp discussed in conjunction with Figures 1A, 1B, 2, 3, 4, and 5. In contrast to the clasp found on those vasooclusive devices, the Figure 9A clasp (160) contains no lumen therethrough for a control wire. As was the case with the clasp above, the clasp is generally cylindrical in shape but has a surface (162) which may be cut or milled away to allow it to mesh with a receiver area (164) in the next adjacent clasp as is shown in Figure 11. The other end of the clasp is adapted to allow joining with the end of a coil or pusher. Although that other end is shown with a reduced diameter (166), to allow insertion of the end (166) into the coil or pusher, other end shapes are certainly appropriate, e.g., helical to accept the coil, square, bulbed, etc., the reduced diameter cylinder is very desirable. The vertical or mating surface (168) pulls the next device in the chain along when it contacts the similar surface in that next device. The end surface (170) pushes against the next device when in a chain.

Figure 9B shows a front, elevated, three-quarter view of the clasp found in Figure 9A. Figure 9B shows the end surface (170), the receiver area (164), and the reduced diameter shaft (166) for mating with the coil or pusher.

Figure 10A shows a clasp (172) much like that shown in Figure 9A except that the end surface is a ramp (174) to permit assembly ease in placing the coils in the catheter or introducer.

Figure 10B shows a front, elevated, three-quarter view of the clasp found in Figure 10A. Figure 10B shows the end ramp (174), the receiver area (164), and the reduced diameter shaft (166) for mating with the coil or pusher.

Figure 11 shows how the clasps found in Figures 9A and 9B mesh when installed on a pusher (178) or a coil (180). It is within the scope of this variation of the invention that the clasp be mounted on the ends of pushers, coils (one or both ends), and that multiple coils or other vasooclusive devices be joined in multiple end-to-end trains for introduction into the vasculature.

## Claims

1. A detachable coil assembly (100,112,128) for use in occluding a selected site within a vessel, comprising a coil (102,116) adapted for detachment in a body cavity and with opposing ends on a coil axis, characterised in that the coil has, on at least one end thereof, an interlockable, shaped clasp (104,114,132,142,160,172) extending in the same general direction as the coil axis, said interlockable clasp being configured to interlock detachably with a similar clasp.

2. The assembly of claim 1, additionally having an axial passageway generally colinear with said coil axis and where said coil assembly may be coupled to said similar clasp by passing a control wire (106) through the axial passageway and where the coil assembly may be uncoupled from said similar clasp by axially withdrawing the control wire from the aligned axial passageways in said clasps.

3. The assembly of claim 1 or claim 2, where the coil assembly (112) has interlockable clasps (114) at each of its coil ends.

4. The assembly of claim 1, 2 or 3, in which the interlockable clasp (104,114) of the coil assembly comprises a distal portion of a generally cylindrical shape, a middle portion (108) adapted to accept said generally cylindrical distal portion from said similar clasp, and a proximal section attached to the coil.

5. The assembly of any one of claims 1 to 3, in which the interlockable clasp of the coil assembly comprises a generally cylindrical distal portion having a ramp (136,174) adapted to engage a ramp on said similar interlockable clasp, a middle portion adapted to accept a cylindrical portion with a ramp of said similar interlockable clasp, and a proximal portion attached to the coil.

6. The assembly of any one of the preceding claims, additionally comprising a control wire (106) extending therethrough.

7. The assembly of claim 6, additionally comprising one or more coil assemblies (100,110,112,128) interlocked by the control wire (106).

8. A combination pusher assembly-coil assembly, comprising a coil assembly (100,112,128) as claimed in any one of the preceding claims and a pusher assembly (118) comprising a tubular pusher sheath (124) having a proximal end and a distal end and adapted to fit within a catheter sheath (120), wherein said similar interlockable clasp (110) is located at the distal end of the pusher sheath and is connected to the distal end of the pusher assembly.

9. The combination pusher assembly-coil assembly of claim 8 when appendant to claim 2, wherein the pusher assembly (118) also has a continuous passageway extending therethrough for passage of the control wire (106) from the distal end of the pusher sheath (124) to the proximal end of the pusher sheath, and the combination further comprises the control wire (106), said control wire being suitable for passage though the pusher passageway and the axial passageway of the coil assembly.

10. The assembly of claim 8 or 9, additionally comprising a catheter sheath (120) disposed about the pusher sheath (124).

11. The assembly of any one of the preceding claims, wherein the coil axis length is 0.5 to 100 cm.

12. The assembly of any one of the preceding claims, wherein the coil outer diameter is between 0.25 mm and 1.8 mm.

13. The assembly of any one of the preceding claims, wherein the coil is a helical coil.

14. The assembly of any one of the preceding claims, where the coil has a random or straight configuration.

15. The assembly of any one of the preceding claims, comprising a plurality of coils.

## Patentansprüche

1. Lösbare Wendelbaugruppe (100, 112, 128) zur Anwendung beim Verschluss eines ausgewählten Situs in einem Gefäß, die eine Wendel (102, 116) aufweist, die zur Freigabe in einer Körperhöhle eingerichtet und mit ihren entgegengesetzten Enden auf einer Wendelachse angeordnet ist, dadurch gekennzeichnet, dass die Wendel wenigstens an einem ihrer Enden eine verriegelbare, geformte Schließe (104, 114, 132, 142, 160, 172) hat, die sich in derselben Hauptrichtung erstreckt wie die Wendelachse und die so gestaltet ist, dass sie lösbar mit einer gleichartigen Schließe verriegelbar ist.

2. Baugruppe nach Anspruch 1, die zusätzlich einen axialen Kanal hat, der sich im wesentlichen kolinear zur Wendelachse erstreckt, wobei die Wendelbaugruppe mit einer gleichartigen Schließe gekoppelt werden kann, indem ein Bediendraht (106) durch den axialen Kanal geschoben wird und die Wendelbaugruppe von der gleichartigen Schließe abgekoppelt werden kann, indem der Bediendraht aus den miteinander fluchtenden axialen Kanälen in den Schießen axial herausgezogen wird.

3. Baugruppe nach Anspruch 1 oder 2, wobei die Wendelbaugruppe (112) an jedem der Wendelenden verriegelbare Schließen (114) hat.

4. Baugruppe nach Anspruch 1, 2 oder 3, bei der die verriegelbare Schließe (104, 114) der Wendelbaugruppe einen distalen Abschnitt in hauptsächlich zylindrischer Form, einen zur Aufnahme des hauptsächlich zylindrischen distalen Abschnitts einer gleichartigen Schließe angepassten mittleren Abschnitt (108) und einen an der Wendel angebrachten proximalen Abschnitt hat.

5. Baugruppe nach einem der Ansprüche 1 bis 3, bei der die verriegelbare Schließe der Wendelbaugruppe einen im wesentlichen zylindrischen distalen Abschnitt, der eine zum Eingriff an einer Schrägfläche einer gleichartigen verriegelbaren Schließe eingerichtete Schrägfläche(136, 174), einen zur Aufnahme eines zylindrischen Abschnitts mit der Schrägfläche der gleichartigen verriegelbaren Schließe eingerichteten mittleren Abschnitt und einen an der Wendel angebrachten proximalen Abschnitt hat.

6. Baugruppe nach einem der vorangehenden Ansprüche, die zusätzlich einen hindurchgehenden Bediendraht (106) aufweist.

7. Baugruppe nach Anspruch 6, die außerdem eine oder mehrere durch den Bediendraht (106) miteinander verriegelte Wendelbaugruppen (100, 110, 112, 128) aufweist.

8. Kombination einer Schiebeanordnung mit einer Wendelbaugruppe, die eine Wendelbaugruppe (100, 112, 128) nach einem der vorangehenden Ansprüche und eine Schiebeanordnung (118) aufweist, welche eine tubusförmige Schiebehülse (124) aufweist, die ein proximales und ein distales Ende hat und dazu angepasst ist, innerhalb einer Katheterhülse (120) zu liegen, wobei die gleichartige verriegelbare Schließe (110) am distalen Ende der Schieberhülse liegt und mit dem distalen Ende der Schiebeanordnung verbunden ist.

9. Kombination einer Schiebeanordnung mit einer Wendelbaugruppe nach Anspruch 8, soweit dieser von Anspruch 2 abhängt, bei der die Schiebeanordnung (118) auch einen sie durchsetzenden kontinuierlichen Kanal hat, für den Durchgang des Bediendrahts (106) vom distalen Ende der Schiebehülse (124) zu ihrem proximalen Ende, und die Kombination außerdem den Bediendraht (106) aufweist, der dazu eingerichtet ist, durch den Kanal der Schiebeanordnung und den axialen Kanal der Wendelbaugruppe hindurchzugehen.

10. Baugruppe nach Anspruch 8 oder 9, die zusätzlich eine Katheterhülse (120) aufweist, die um die Schieberhülse (124) angeordnet ist.

11. Baugruppe nach einem der vorangehenden Ansprüche, bei der die Länge der Wendelachse 0,5 bis 100 cm ist.

12. Baugruppe nach einem der vorangehenden Ansprüche, bei der der Außendurchmesser der Wendel zwischen 0,25 mm und 1,8 mm liegt.

13. Baugruppe nach einem der vorangehenden Ansprüche, bei der die Wendel helixförmig ist.

14. Baugruppe nach einem der vorangehenden Ansprüche, bei der die Wendel eine zufällige oder geradlinige Gestalt hat.

15. Baugruppe nach einem der vorangehenden Ansprüche, die mehrere Wendeln aufweist.

## Revendications

1. Ensemble de serpentin détachable (100, 112, 128) destiné à être utilisé pour occlure un site sélectionné à l'intérieur d'un vaisseau, comprenant un serpentin (102, 116) adapté pour être détaché dans une cavité corporelle et avec des extrémités opposées sur un axe de serpentin, caractérisé en que le serpentin comporte, sur au moins une extrémité de celui-ci, un crampon profilé verrouillable (104, 114, 132, 142, 160, 172) s'étendant dans la même direction générale que l'axe du serpentin, ledit crampon verrouillable étant configuré de façon à se verrouiller de façon détachable avec un crampon similaire.

2. Ensemble selon la revendication 1, comportant de plus un passage axial globalement colinéaire avec ledit axe de serpentin, et dans lequel ledit ensemble de serpentin peut être couplé audit crampon similaire en faisant passer un fil de commande (106) à travers le passage axial, et dans lequel l'ensemble de serpentin peut être découplé dudit crampon similaire par retrait axial du fil de commande des passages axiaux alignés dans lesdits crampons.

3. Ensemble selon la revendication 1 ou la revendication 2, dans lequel l'ensemble de serpentin (112) comporte des crampons verrouillables (114) à chacune de ses extrémités de serpentin.

4. Ensemble selon la revendication 1, 2 ou 3, dans lequel le crampon verrouillable (104, 114) de l'ensemble de serpentin comprend une partie distale de forme globalement cylindrique, une partie moyenne (108) adaptée pour recevoir ladite partie distale globalement cylindrique dudit crampon similaire, et une section proximale fixée au serpentin.

5. Ensemble selon l'une quelconque des revendications 1 à 3, dans lequel le crampon verrouillable de l'ensemble de serpentin comprend une partie distale globalement cylindrique comportant une rampe (136, 174) adaptée pour venir en prise avec une rampe sur ledit crampon verrouillable similaire, une partie médiane adaptée pour accepter une partie cylindrique avec une rampe dudit crampon verrouillable similaire, et une partie proximale fixée au serpentin.

6. Ensemble selon l'une quelconque des revendications précédentes, comprenant de plus un fil de commande (106) s'étendant à travers celui-ci.

7. Ensemble selon la revendication 6, comprenant de plus un ou plusieurs ensembles de serpentin (100, 110, 112, 128) verrouillés par le fil de commande (106).

8. Combinaison ensemble de poussoir-ensemble de serpentin, comprenant un ensemble de serpentin (100, 112, 128) selon l'une quelconque des revendications précédentes et un ensemble de poussoir (118) comprenant un manchon de poussoir tubulaire (124) comportant une extrémité proximale et une extrémité distale et adapté pour s'engager à l'intérieur d'un manchon de cathéter (120), dans lequel ledit crampon verrouillable similaire (110) est disposé à l'extrémité distale du manchon de poussoir et est raccordé à l'extrémité distale de l'ensemble de poussoir.

9. Combinaison ensemble de poussoir-ensemble de serpentin selon la revendication 8 lorsqu'elle dépend de la revendication 2, dans laquelle l'ensemble de poussoir (118) comporte également un passage continu s'étendant à travers celui-ci pour le passage du fil de commande (106) de l'extrémité distale de manchon de poussoir (124) à l'extrémité proximale du manchon de poussoir, et la combinaison comprend de plus le fil de commande (106), ledit fil de commande étant approprié pour passer à travers le passage de poussoir et le passage axial de l'ensemble de serpentin.

10. Ensemble selon la revendication 8 ou 9, comprenant de plus un manchon de cathéter (120) disposé autour du manchon de poussoir (124).

11. Ensemble selon l'une quelconque des revendications précédentes, dans lequel la longueur de l'axe de serpentin est comprise entre 0,5 et 100 cm.

12. Ensemble selon l'une quelconque des revendications précédentes, dans lequel le diamètre extérieur du serpentin est compris entre 0,25 mm et 1,8 mm.

13. Ensemble selon l'une quelconque des revendications précédentes, dans lequel le serpentin est un serpentin hélicoïdal.

14. Ensemble selon l'une quelconque des revendications précédentes, dans lequel le serpentin a une configuration aléatoire ou droite.

15. Ensemble selon l'une quelconque des revendications précédentes, comprenant une pluralité de serpentins.
